# EUROPEAN PATENT APPLICATION

(11) **EP 2 189 456 A1**
(43) Date of publication of application: **26.05.2010**
(21) Application number: 08169161.0
(22) Date of filing: 14.11.2008
(51) Int. Cl.: C07D 401/04

(54) **New compounds prepared from omeprazole**

(71) Applicant: LEK Pharmaceuticals d.d., 1526 Ljubljana (SI)
(72) Inventor: Grahek, Rok, 1526 Ljubljana (SI); Bastarda, Andrej, 1526 Ljubljana (SI); Kocijan, Andrej, 1526 Ljubljana (SI)

(57) **Abstract**

New compounds, which are prepared by using omeprazole as a starting substance, salts of these compounds, as well their use as impurity standards are provided. Furthermore, the invention relates to a process for the preparation of these compounds, and a method for analyzing a sample of omeprazole.

## Description

The present invention relates to new compounds which are prepared from omeprazole, salts of these compounds, as well the use of these compounds as impurity standards. Furthermore, the invention relates to a process for the preparation of these reaction products of omeprazole, and a method for analyzing a sample of omeprazole.

All drugs contain impurities. Organic impurities can origin from raw materials, reagents and intermediates used in synthesis or bio-synthesis of drug or they can be synthetic or biosynthetic by-products or degradation products. It is important to know that impurities can affect safety and also efficacy of the drug. Therefore a high level of purity of drug substance and knowing the impurity profile are important criterions in manufacturing of a safe and efficient drug. In general, drug impurities in excess of 0.1% should be identified and quantified by selective methods.

The substance 5-methoxy-2(((4-methoxy-3,5-dimethyl-2-pyridinyl)methyl)sulfinyl)-1H-benzimidazole, known under the generic name omeprazole, is described in EP-A 0 005 129.

Omeprazole is useful for inhibiting gastric acid secretion and has gastric mucosa protective activity. Accordingly, omeprazole may be used for prevention and treatment of gastric acid related disorders in mammals and man, including e.g. gastro esophageal reflux disease, gastritis, gastric ulcer and duodenal ulcer.

Omeprazole is susceptible to degradation/transformation in acid reacting and neutral media. The half-life of degradation of omeprazole in water solutions at pH-values <4 is shorter than 10 minutes. Also at neutral pH-values degradation proceeds rapidly, e.g. at pH 7 the half-life of omeprazole is about 14 hours, while at higher pH-values the stability in solution is much better (Pilbrant and Cederberg, Scand. J. Gastroenterology 1985; 20 (suppl. 108) p. 113-120).

WO 2005/056534 A1 discloses degradation products of the statin compound rosuvastatin as well as their use as a reference standard (including reference marker) for the analysis of rosuvastatin.

However, none of the cited prior art describes the compounds as disclosed in the invention.

The object of the present invention is the provision of two new compounds. In particular, it is an aim of the invention to provide two compounds which can be used as impurity standard for analysis of a finished dosage form (FDF) of omeprazole, e.g. omeprazole capsules, and which give the opportunity to complete the characterization of impurity (response factor, toxicological studies).
This object is solved by the features of the main claims. Preferred embodiments of the invention are defined in the dependent claims.

According to a first aspect, the invention provides a compound (OMP-A) having the formula 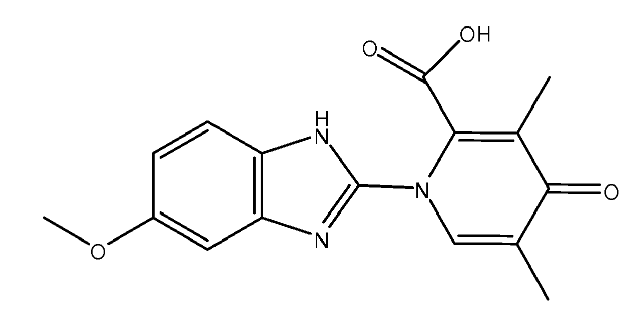 or a salt thereof.

According to a second aspect, the invention provides a compound (OMP-AEt) having the formula 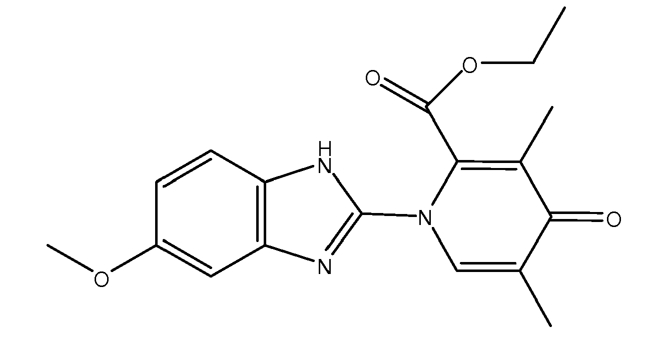 or a salt thereof.

Furthermore, the invention provides processes for the preparation of the above compounds as well as a method for analyzing a sample of omeprazole and a method for determining the retention time on a chromatographic system of omeprazole using these compounds.

### Summary of the invention

The new compounds are formed by the reaction of omeprazole.

The first substance (OMP-A) may be formed by the reaction of omeprazole in neutral or acidic aqueous media. It can also be formed, to a lesser extent, by the reaction of solid (crystalline) omeprazole in the presence of water.

The second substance (OMP-AEt) may be formed from OMP-A in anhydrous media in the presence of ethanol. It can also be formed, to a lesser extent, by the reaction of solid (crystalline) omeprazole in the presence of ethanol.

The new compounds have been spectroscopically characterized by using Mass Spectrometry (MS) and Nuclear Magnetic Resonance (NMR) spectroscopy.

The new compounds may be in the form of a salt. Salts of compounds having salt-forming groups are especially acid addition salts, salts with bases or, where several salt-forming groups are present, can also be mixed salts or internal salts. Salts are especially selected from the group consisting of salts from alkali metals, earth alkali metals and ammonium. Preferably, the salts are selected from salts of sodium, magnesium and ammonium.

The invention is also directed to processes for the preparation of the compounds.

Preferably, the substance OMP-A can be prepared by a process comprising the steps of:
a) contacting omeprazole with an aqueous medium and/or water;
b) isolating OMP-A.

In a particularly preferred process for the preparation of OMP-A, the reaction mixture is preferably constituted of 1% (w/v) omeprazole, 50% (v/v) 5-500 mM, more preferably about 50 mM phosphate buffer, the pH is set in a range of 2-7, more preferably 3-4, and 50% (v/v) acetonitrile. The reaction mixture may be set in a range of room temperature to reflux temperature, preferably to a temperature of about 75 °C, stirred at that temperature for 0.1 to 24 hours, more preferably about 1 hour, and left at room temperature for the next 0.1 to 120 hours, more preferably about 20 hours. The reaction mixture may be then concentrated by evaporation under reduced pressure. The obtained solution is preferably neutralized by addition of aqueous solution of sodium hydroxide to a pH in the range of 6 to 14, more preferably to a pH in the range of 7 to 8. The clear solution is preferably decanted and may be further concentrated by evaporation under reduced pressure. Afterwards, OMP-A is preferably isolated from the obtained solution by preparative HPLC by preferably using a suitable reverse phase column.

Preferably, the substance OMP-AEt can be prepared by a process comprising the steps of:
a) contacting omeprazole and/or OMP-A with ethanol; and
b) isolating OMP-AEt.

In a particularly preferred process for the preparation of OMP-AEt, OMP-A is sealed in a container, preferably a glass vial, together with another container containing dry molecular sieves and absolute ethanol. The sealed container is preferably thermostated at 20 to 80°C, more preferably about 60°C for 5 to 48, more preferably about 24 hours. OMP-AEt may be isolated from the obtained reaction mixture by preparative HPLC using a suitable reverse phase column.

The invention is also directed to a method for analyzing a sample of omeprazole comprising the steps of:
a) performing chromatography on the sample to obtain data; and
b) comparing the data with the chromatography data of the above compounds or salts thereof.

As the chromatography method any suitable method for the chromatographic separation of mixtures comprising omeprazole and/or the compounds of the invention may be used

Preferably, this method may comprise the following steps:
(a) preparing a solution of omeprazole containing at least one of the above compounds or a salt thereof;
(b) subjecting the solution to a high pressure liquid chromatography to obtain a chromatogram; and
(c) comparing the peaks obtained in the chromatogram to a peak resulting from at least one of the above compounds or a salt thereof.

Alternatively, this method may comprise the following steps:
(a) preparing a solution of omeprazole containing at least one of the above compounds;
(b) subjecting the solution to thin layer chromatography to obtain a chromatogram; and
(c) comparing the bands or spots obtained in the chromatogram to a peak or band resulting from at least one of the above compounds or a salt thereof.

Furthermore, the invention is directed to a method for determining the retention time of a chromatography column for omeprazole, comprising the steps of carrying out chromatography with at least one of the above compounds, or a salt thereof, as a standard, followed by determining the retention time of omeprazole on the basis of the retention time of at least one compound of the above compounds.

As described above, the present invention provides new compounds which are formed from omeprazole. These compounds can be used as a reference standard for analysis of omeprazole. That is, the isolated compound may be used as an impurity standard for the purposes of quality control analyses and stability of the finished product.

In general, side products, byproducts and adjunct reagents (herein sometimes called impurities) are identified by physical methods and the impurities are associated with a peak position in a chromatogram and/or a spot on a TLC plate. Thereafter, the impurity can be identified by its position in the chromatogram, which is usually measured as the time period between the injection of the sample on a column and the elution of the particular component as detected by a detector, which is the retention time. This time period varies based upon the condition of the instrumentation and other factors. In order to diminish the effect of such variations, the relative retention time (RRT) is used to identify impurities. The RRT of an impurity is its retention time divided by the retention time of a reference marker.

Since pure omeprazole may be present in such high proportion in a mixture of substances that it tends to saturate a column, which may lead to irreproducible retention times. Thus, one or more alternative compound may be selected that is added to, or is present in, the mixture in an amount significant enough to be detected and sufficiently low as not to saturate the column. Accordingly, at least one of the novel compounds of the invention may serve as reference markers. In this connection, the person skilled in the art understands that a compound in a relatively pure state can be used as a reference standard to quantify the amount of the compound in an unknown mixture.

A reference marker is similar to a reference standard but it is used for qualitative analysis. When the compound is used as an external standard, a solution of a known concentration of the compound is analyzed by the same technique as the unknown mixture. As a result, the amount of the compound in the mixture can be determined by comparing the magnitudes of the detector response for the respective compounds.

The reference standard compound also can be used to quantify the amount of another compound in the mixture if the "response factor", which compensates for differences in the sensitivity of the detector to the two compounds, has been predetermined. For this purpose, the reference standard compound may be added directly to the mixture as an internal standard.

The reference standard compound can even be used as an internal standard when the unknown mixture contains some of the reference standard compound by using a technique called "standard addition", wherein at least two samples are prepared by adding known and differing amounts of the internal standard. The proportion of the detector response due to the reference standard compound that is originally in the mixture can be determined by extrapolation of a plot of detector response versus the amount of the reference standard compound that was added to each of the samples to zero. In this connection reference is made to Strobel, H. A.; Heineman, W. R., Chemical Instrumentation: A Systematic Approach, 3rd dd. (Wiley & Sons: New York 1989); and Snyder, L. R.; Kirkland, J. J. Introduction to Modern Liquid Chromatography, 2nd ed. (John Wiley & Sons: New York 1979).

The novel compounds according to the invention, which are prepared and isolated by the methods as described above were structurally characterized by Mass Spectrometry (MS) and Nuclear Magnetic Resonance (NMR) spectroscopy in order to determine the chemical structure of said novel compounds. The methods of characterization and their results are presented in the examples described below.

Using P-HPLC, new compounds were isolated and their structure was determined with the help of MS and NMR measurements. For the characterization of the new compounds mass spectrometry (MS) and Nuclear Magnetic Resonance (NMR) analyses were carried out.

In the following, the invention is further explained by using examples. However, these examples are for illustrative purposes only and should not be interpreted in a restrictive way.

### Examples

### 1. Preparation of OMP-A

A reaction mixture, constituted of 20 g omeprazole, 2 I of 50 mM phosphate buffer, pH 3.6 and 2 I of acetonitrile, was prepared. The above mentioned reaction mixture was heated to 75 °C, stirred at that temperature for one hour and left at room temperature for the next 20 hours. The reaction mixture was then concentrated by evaporation under reduced pressure to approximately 1.5 I. The obtained solution was neutralized by addition of aqueous solution of sodium hydroxide to a pH between 7 and 8. The clear solution was decanted and further concentrated by evaporation under reduced pressure to approximately 1 I. OMP-A was isolated from the obtained solution by preparative HPLC.

Chromatographic conditions of the preparative separation:
Column: Luna 10µm prep C18(2) (200 x 50) mm
Mobile phase A: 10 mM ammonium hydrogen carbonate
Mobile phase B: Acetonitrile
Flow rate: 140 ml/min
Gradient:

| Time (min) | % B |
|---|---|
| 0 | 2 |
| 0.1 | 2 |
| 1 | 15 |
| 4.5 | 15 |
| 5 | 90 |
| 8.85 | 90 |
| 9 | 2 |

Sample load: 80 ml
Detector: UV, 280 nm

A fraction was collected from 3^{rd} to 4^{th} minute. The collected fraction was concentrated by evaporation at reduced pressure. The final product was dried in vacuum. 4.9g of the pure final product was obtained.

### 2. Preparation of OMP-AEt

100 mg of OMP-A was sealed in a 10 ml glass vial, together with a smaller 2 ml vial, containing dry molecular sieves and absolute ethanol. The sealed vial was thermostated at 60 °C for 24 hours. OMP-AEt was isolated from the obtained reaction mixture by preparative HPLC.

Chromatographic conditions of the preparative separation:
Column: Kromasil Si 7µm, (250 x 10) mm
Mobile phase A: n-hexane
Mobile phase B: dichloromethane
Mobile phase C: ethanol abs.
Flow rate: 10 ml/min
Gradient:

| Time (min) | %B | % C |
|---|---|---|
| 0.0 | 30 | 5 |
| 0.6 | 30 | 5 |
| 0.8 | 30 | 8 |
| 4.0 | 30 | 11 |
| 4.5 | 20 | 70 |
| 6 | 20 | 70 |

Sample load: 1 ml
Detector: UV, 320 nm

A fraction was collected and concentrated by evaporation at reduced pressure. The final product was dried in vacuum. 56mg of the pure final product was obtained.

### 3. Mass spectrometry measurements of the novel compounds prepared from omeprazole FDF

Mass spectrometric measurements were performed with Finnigan TSQ 7000 instrument (San Jose, CA, USA) interfaced with an Atmospheric Pressure Chemical Ionization (APCI) ion source.

The APCI source parameters were: vaporiser temperature set to 520ºC, capillary temperature 220ºC, sheat gas (nitrogen) 90 psi, auxiliary gas (nitrogen) 5 mL·min⁻¹, corona 4 µA. Sample was introduced with loop injection 5 µL. Mobile phase (10 mM ammonium acetate, 50% acetonitrile) flow rate was 1 mL·min⁻¹.

By MS/MS measurements Argon (2.5 mTorr) was used as collision gas. Collision energy was set to 20 V.

Reaction product OMP-A has (M+H)⁺ m/z 314. Molecular mass of reaction product OMP-A is 313 Da.

Reaction product OMP-AEt has a (M+H)⁺ m/z 342. Also an adduct ion with acetonitrile could be observed (M+H+MeCN)⁺ m/z 383. Molecular mass of reaction product OMP-AEt is 341 Da.

### 4. NMR measurements of novel compounds prepared from omeprazole FDF

The structure of the reaction products OMP-A and OMP-AEt were confirmed with ¹H and ¹³C NMR studies.

OMP A was prepared in CD₃OD. ¹H analyses were performed on Varian INOVA 300 NMR spectrometer. As the reference residual signal of CD₃OD (3.31 ppm) was used. ¹³C data were obtained from analyses on Varian VNMRS 400 instrument. As the reference residual signal of CD₃OD (49.15 ppm) was used.

OMP AEt was prepared in CDCl₃. ¹H analyses were performed on Varian INOVA 300. As the reference residual signal of CDCl₃ (7.27 ppm) was used.

### Structures of OMP-A and OMP-AEt

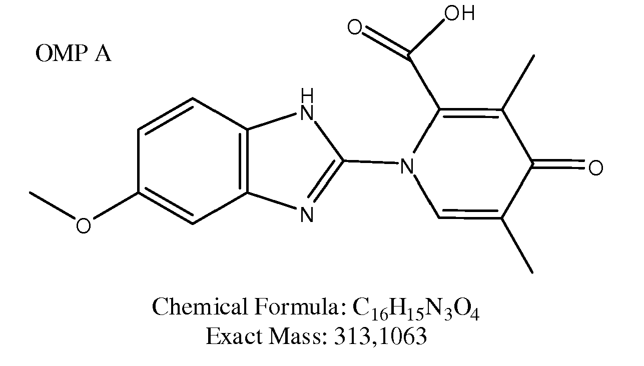

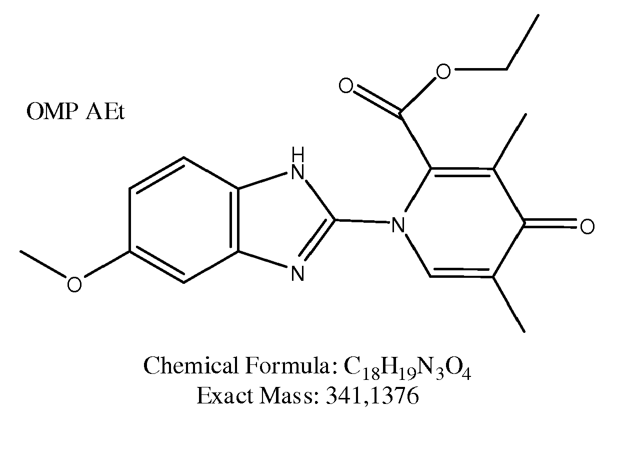

## Claims

1. A compound having the formula 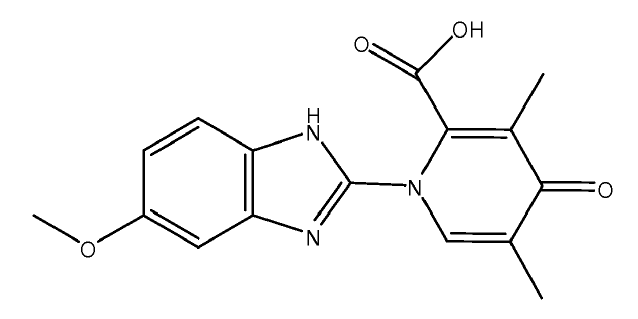 or a salt thereof.

2. A compound having the formula 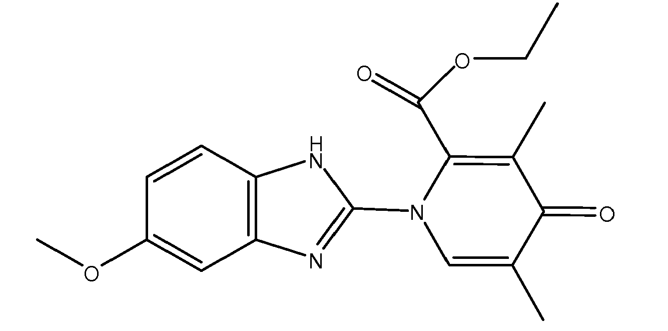 or a salt thereof.

3. The compound according to claim 1 or claim 2, wherein the salt is selected from the group consisting of salts of earth alkali metals, alkali metals and ammonium, preferably salts of sodium, magnesium or ammonium.

4. The use of a compound according to Claim 1 or 2, or a salt thereof as an impurity standard.

5. A process for the preparation of the compound according to claim 1, comprising the steps of:
a) contacting omeprazole with an aqueous medium and/or water; and
b) isolating OMP-A.

6. A process for the preparation of the compound according to claim 2, comprising the steps of:
a) contacting omeprazole and/or the compound according to claim 1 with ethanol; and
b) isolating OMP-AEt.

7. A method for analyzing a sample of omeprazole comprising the steps of:
a) performing chromatography on the sample to obtain data; and
b) comparing the data with the chromatography data of the compound of claim 1 and/or 2.

8. The method of claim 7, wherein the method comprises the following steps:
(a) preparing a solution of omeprazole containing at least one of the compounds of claim 1 and/or 2;
(b) subjecting the solution to a high pressure liquid chromatography to obtain a chromatogram; and
(c) comparing a peak obtained in the chromatogram to a peak resulting from the compound of claim 1 and/or 2.

9. The method of claim 7, wherein the method comprises the following steps:
(a) preparing a solution of omeprazole containing the compound of claim 1 and/or 2;
(b) subjecting the solution to thin layer chromatography to obtain a chromatogram; and
(c) comparing a band or spot obtained in the chromatogram to a peak or band resulting from the compound of claim 1 and/or 2.

10. A method for determining the retention time on a chromatographic system for omeprazole, comprising the steps of:
carrying out chromatography with at least one compound of claim 1 and/or 2 as a standard, and
determining the retention time of omeprazole on the basis of the retention time of at least one compound of claim 1 and/or 2.
